# EUROPEAN PATENT SPECIFICATION

(11) **EP 0 281 037 B1**
(45) Date of publication and mention of the grant of the patent: **26.05.1993**
(21) Application number: 88102928.4
(22) Date of filing: 26.02.1988
(51) Int. Cl.: G01N 33/00

(54) **Method of measuring oxygen in silicon**
Verfahren zur Messung des Sauerstoffsgehaltes in Silizium
Méthode de mesure de l'oxygène dans le silicium

(30) Priority: 28.02.1987 JP 47086/87
(43) Date of publication of application: 07.09.1988
(73) Proprietor: HORIBA, LTD., Minami-ku Kyoto (JP)
(72) Inventor: Tsuji, Katsuya, Hyogo-ku Kobe-City Hyogo-prefecture (JP); Yamada, Takeshi, Ohtsu-City Shiga-prefecture (JP); Uchihara, Hiroshi Nagitsuji Nishi-shieijutaku3-104, Yamashina-ku Kyoto (JP)
(74) Representative: TER MEER STEINMEISTER & PARTNER GbR

(56) References cited:
- FR-A- 2 105 800
- GB-A- 2 184 235
- US-A- 3 946 228
- US-A- 4 098 576

## Description

### BACKGROUND OF THE INVENTION

### Field of the Invention

The present invention relates to a method of measuring the quantity of oxygen contained in silicon, in particular to a quite novel method of measuring oxygen in silicon whereby a handy, speedy and accurate control analysis of highly pure single crystalline silicon which is a constituent ingredient in the manufacturing process of for example a semi-conductor silicon wafer can be carried out.

### Description of the Prior Art

As methods of measuring oxygen in silicon, methods of measuring absolute quantities, such as an activation analysis using charged particles and a vacuum-fusion method and methods of measuring relative quantities, such as a secondary ion mass analysis and an infrared absorption analysis, are previously known.

Among these various kinds of methods, the former three methods require remarkably large-scaled and expensive facilities or a long measuring time and cause therefore much trouble. Accordingly, they are unsuitable for industrial control analysis and hardly used practically. Therefore, they have been used merely for the analysis on an investigation level. Accordingly, in most cases, the infrared absorption analysis merely requiring a relatively handy apparatus and measuring operation has been used as industrially practical control analysis.

However, said infrared absorption analysis comprises the following problems:
Since the infrared absorption analysis is a method of measuring a relative quantity, the accuracy of measurement suffers in that a relatively large error of measurement is caused by the thickness-effect of the silicon sample (i.e., when the thickness of the silicon sample is not strictly equal to that of the standard sample). But it is also usual with infrared ray-opaque silicon samples, such as a silicon wafer doped with phosphor, boron, antimony and the like, which has been recently developed that the fatal defect occurs in that the infrared absorption analysis cannot be applied at all. Accordingly, when such an infrared ray-opaque silicon sample is to be measured, the investigating method, such as said secondary ion mass analysis, which is remarkably disadvantageous in economy, measuring efficiency (usually about 1 hour/1 analysis), operation and the like, cannot but be used in place of the infrared absorption analysis.

The present inventors carried out various studies in the field of development of a method of measuring oxygen in silicon which can be carried out as a more handy practical control analysis in place of the conventional infrared absorption analysis and which is sufficiently applicable even when an infrared ray-opaque silicon sample is to be measured.

As a result, a method of measuring oxygen in silicon using an extraction analysis of gases in a metal by the heat melting method has been found.

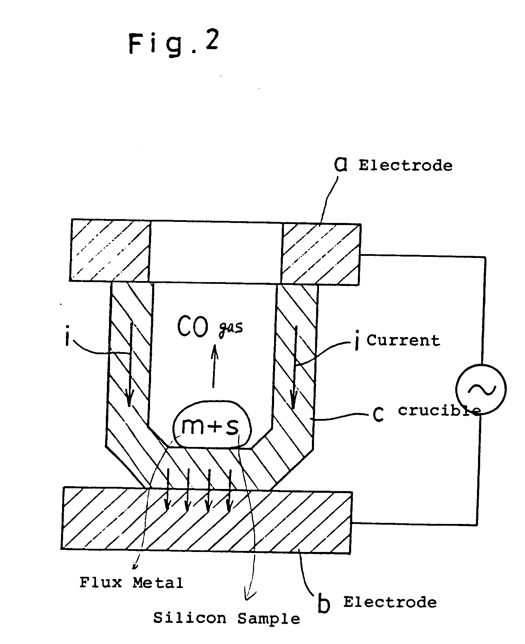

A prior art method of measuring oxygen in silicon is roughly shown in Fig. 2 wherein a graphite crucible c of which the temperature can be adjusted by putting it between an electrode a and an electrode b in a pressed manner and electrifying it (by flowing an electric current i through it) to generate a heat, is at first heated at a predetermined high temperature to degas the graphite crucible c itself. Then a silicon sample s is charged together with a flux metal m into the graphite crucible c (for example a metal, such as nickel and tin, for the use in a metallic bath) to extract oxygen contained in the silicon sample s in form of gases combined with carbon (for example carbon monoxide gas). Subsequently, the extracted gases are introduced into a conventional gas-concentration analyzer system (not shown) to detect the gas concentration, whereby measuring the quantity of oxygen contained in said silicon sample s, i.e. the same procedure as for the measurement of for example iron is adopted.

The method of measuring oxygen in silicon using said heat melting type gas extraction analysis can be principally also applied to an infrared ray-opaque silicon sample and can very easily measure a quantity of oxygen contained in the silicon sample. However, various kinds of problems do arise:
(a) The following reaction takes place between the graphite crucible c and the silicon sample s charged into said graphite crucible c to locally produce deteriorated portions (SiC portions having a large electric restistance) in the graphite crucible c, so that a remarkably large amount of Joule's heat is generated in the deteriorated portions by the electric current i flowing through the graphite crucible c to locally heat the graphite crucible c to an abnormally high temperature:

   C + Si → SiC

   Accordingly, a reaction takes place between oxygen (so called metal oxide) contained in the graphite crucible c itself and a part of said SiC as follows:

   SiC + O → Si + CO

   As a result, blank gas (carbon monoxide gas) is generated leading to a great error of measurement and the graphite crucible c is injured.
(b) Since also oxygen contained in the flux metal m itself is simultaneously extracted during heating the silicon sample s, a highly pure flux metal m containing a remarkably small amount of oxygen must be used. This leads also to a great error of measurement.
(c) An error of measurement can be also caused by the rapid evaporation of the silicon sample s within the graphite crucible c and the getter effect of the gas (carbon monoxide gas) extracted from the vaporized silicon.

Therefore, practically good results could not be obtained.

### SUMMARY OF THE INVENTION

It is object of the present invention to eliminate the above described problems in view of the above described prior art methods. It is therefore object of the present invention to provide a method of measuring oxygen in silicon which can applied even to an infrared ray-opaque silicon sample to be measured. With this method the quantity of oxygen contained in the silicon sample can be easily and speedily measured with a sufficiently high accuracy, which method is suitably utilized in simplified industrial control analysis.

The invention relates to a method of measuring the quantity of oxygen contained in a silicon sample comprising
(i) heating the double graphite crucible C consisting of an outside graphite crucible 9 of which the temperature is controlled by electrifying to generate heat and an inside graphite crucible 10 provided in said outside graphite crucible of which the temperature is indirectly controlled at first at a predetermined high temperature to degas said double graphite crucible itself;
(ii) adusting the temperature of said double graphite crucible at a predetermined value and charging a flux metal M into said inside graphite crucible to degas said flux metal;
(iii) adjusting the temperature of said double graphite crucible at values near the melting point of silicon and charging a silicon sample S into said inside graphite crucible to melt said silicon sample in the flux metal and then adjusting the temperature of said double graphite crucible at values which are relatively higher than the melting point of the silicon to extract oxygen contained in said silicon sample in form of gases combined with carbon; and
(iv) introducing said extracted gases into a gas-concentration analyzing system to detect the gas-concentration, whereby measuring the quantity of oxygen contained in said silicon sample.

Basically, the conventional heat melting type gas extraction analysis capable of easily and speedily measuring in the above described manner is applied to the above described method of measuring oxygen in silicon according to the present invention. However, the conventional single type graphite crucible is not used whereas a double graphite crucible is used comprising an outside graphite crucible of which the temperature can be directly adjusted by electrifying to generate heat (through which an electric current flows), and an inside graphite crucible provided in said outside graphite crucible of which the temperature is indirectly adjusted (through which an electric current hardly flows). The silicon sample is charged into the inside graphite crucible. Accordingly, problem (a) occuring by using the above described single type graphite crucible (i.e., generation of blank gas and injury of crucible) is no more relevant. Even though the following reaction takes place between the inside graphite crucible and the silicon sample to locally form deteriorated portions (SiC portions), an electric current hardly flows through the inside graphite crucible, so that the inside graphite crucible is not heated locally to an abnormally high temperature as it is the case with said single type graphite crucible:

C + Si → SiC

In addition, since not only the double graphite crucible itself but also the flux metal is degassed before the silicon sample is charged into the inside graphite crucible, even though a particularly highly pure flux metal is not used, oxygen contained in the flux metal itself can be surely prevented from leading to an error of measurement, so that even if a metal of usual grade is used, said problem (b) can be also solved. Accordingly, the method of measuring oxygen in silicon according to the present invention is economically advantageous.

In addition, the silicon sample is charged into said double graphite crucible under the condition that the temperature of said double graphite crucible is adjusted at values near the melting point of silicon to melt the silicon sample in said flux metal and then the temperature of said double graphite crucible is raised to values which are relatively higher than the melting point of silicon, so that the temperature of the double graphite crucible during the extraction of gases is controlled in two stages, whereby the error of measurement due to the rapid evaporation of the silicon sample and the getter effect accompanied by said rapid evaporation of the silicon sample hardly occurs, whereby also the above described problem (c) has been eliminated.

Thus, the synergistic action of the above described effects leads to a process which can be carried out very easily and speedily providing a very highly accurate measurement (with a time spent which is about 1/10 to 1/20 times than that as required in secondary ion mass analysis).

### BRIEF DESCRIPTION OF THE DRAWINGS

The invention will be explained by means of the accompanying figures.
- **Fig 1 (a) to (c)**: show a flow chart illustrating the procedure; and
- **Fig. 1 (d) to (f)**: show a graph illustrating the temperature within the crucible, the quantity of carbon monoxide generated and the time history of detection output, respectively.
- **Fig.2**: shows a diagram of the conventional heat melting type gas extraction analysis for describing the problems of the prior art and the technical background of the present invention.

### DESCRIPTION OF THE PREFERRED EMBODIMENT

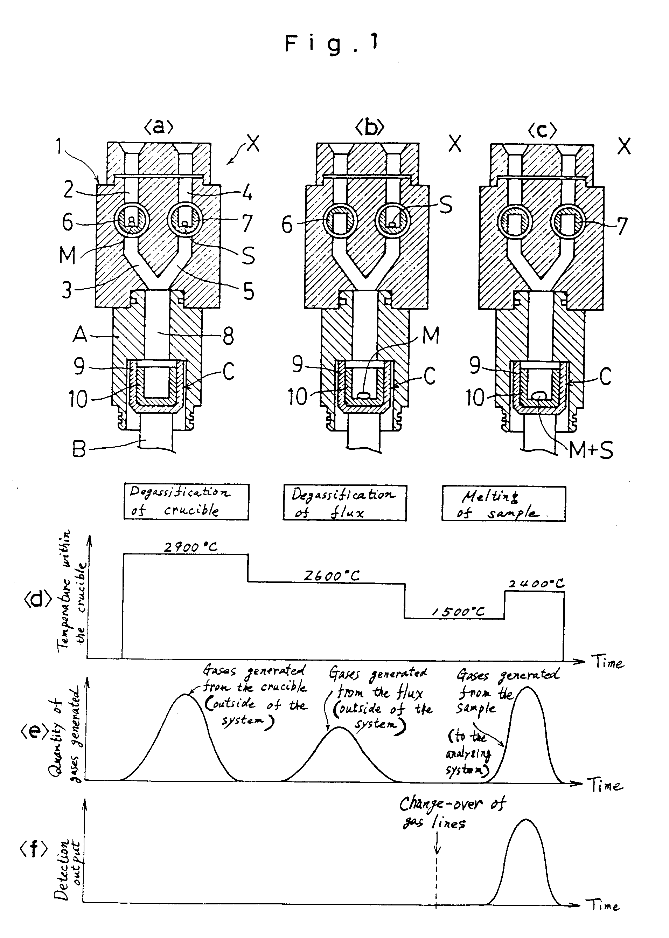

One concrete preferred embodiment of the method of measuring oxygen in silicon according to the present invention will be described below with reference to Figs. 1(a) to (c), which is a flow chart showing the method of the invention. Figs 1 (d) to (f) comprise each a graph showing the relation between the temperature within the crucible, quantity of carbon monoxide generated and time history of detected output and the time.

In addition, Figs. 1(a) to (c) show a heat melting type gas extraction apparatus portion, which is a principal part of the system of measuring oxygen in silicon.

Said apparatus is designated by X. Reference numeral 1 designates a base provided with a port 2 for charging a flux metal M, a drop passage 3, a port 4 for charging a silicon sample S, a drop passage 5, a hold/drop change-over member 6 between said port 2 and said drop passage 3, and a hold/drop change-over member 7 between said port 4 and said drop passage 5 (although a rotary type hold/drop change-over member is used in this example, another member having a different construction, such as a slide type one, may be used as well). Reference mark A designates an electrode mounted on the lower side of said base 1, said electrode A being provided with a drop passage 8 communicating with said both drop passages 3, 5, said drop passage 8 being provided with a double graphite crucible C at the lower end portion thereof so as to be put between said electrode A and the other electrode B in a pressed manner. This double graphite crucible C consists of an outside graphite crucible 9, of which the temperature can be directly adjusted by flowing an electric current between said both electrodes A, B to generate heat, and an inside graphite electrode 10 provided in said outside graphite crucible 9, of which the temperature can be indirectly adjusted. In short, the outside graphite crucible 9 is electrified, whereas the inside graphite crucible 10 is hardly electrified.
The procedure of the method according to the present invention for measuring oxygen in silicon by using the above desribed heat melting type gas extraction apparatus portion X is now described.
(i) At first, as shown in Fig. 1(a), an electric current flows between said both electrodes A, B under the condition that the double graphite crucible C is empty, i.e., that not only the flux metal M but also the silicon sample S is not charged into the double graphite crucible C, to heat the whole double graphite crucible C at a predetermined high temperature (a temperature of about 2,800 to 3,000°C is suitable, as shown in Fig. 1(d)), thereby degassing the double graphite crucible C itself. Thus, as shown in Fig. 1(e), oxygen, which was contained in the double graphite crucible C itself, is extracted from the inside graphite crucible 10 in form of carbon monoxide gas. This carbon monoxide gas is not introduced into the analyzing system (not shown here) but it is charged from the system as it is, so that, as shown from Fig. 1(f). It is not detected.
(ii) Subsequently, the double graphite crucible C is adjusted so as to lower the temperature thereof until a predetermined value (a temperature of about 2,500 to 2,700°C is suitable, as shown in Fig. 1(d)) and then, as shown in Fig. 1 the flux metal M (nickel of 0.3 to 1.5 g and tin of 0 to 2.0 g for the silicon sample of S of 0.1 to 0.7 g, preferably nickel of 0.5 g and tin of 1.0 g for the silicon sample S of about 0.3 g) is charged into the inside graphite crucible 10 by setting in operation a rotary member 6 to heat and melt the flux metal M, whereby degassing the flux metal M. Thus, as shown in Fig. 1(e), oxygen which was contained in the flux metal M, is extracted in the inside graphite crucible 10 in form of carbon monoxide gas, but also this carbon monoxide gas is discharged from the system without being introduced into the analyzing system, so that, as shown in Fig. 1(f), it is not detected.
(iii) Successively, the double graphite crucible C is adjusted so as to lower the temperature thereof until values near the melting point of silicon (as shown in Fig. 1(d), a temperature of about 1,400 to 1,600°C is suitable])and then, as shown in Fig. 1(c), the silicon sample S (0.3 g in the above described manner) is charged into the inside graphite crucible 10 by setting in operation said changeover member 7 to melt the silicon sample S in said flux metal M. Subsequently, the double graphite crucible C is adjusted so as to rise the temperature thereof until values relatively higher than the melting point of silicon (as shown in Fig. 1(d), a temperature of about 2,300 to 2,500°C is suitable), whereby extracting oxygen contained in said silicon sample S in form of carbon monoxide.
(iv) Thus, as shown in Fig. 1(e), oxygen, which as contained in the silicon sample S, is extracted in the inside graphite crucible 10 in form of carbon monoxide gas (sample gas). The extracted carbon monoxide gas is introduced into a gas-concentration analyzer and a heat-conductivity meter (which are not shown because they are of conventional types) such as a non-dispersive type infrared analyzer and the like, by changing-over gas lines, to detect the concentration of carbon monoxide whereby measuring the quantity of oxygen contained in said silicon sample S.

In addition, said carbon monoxide may be additionally oxidized into carbon dioxide to be measured. This can be suitably arranged in connection with the analyzer.

According to the method of measuring oxygen in silicon of the present invention, a superior effect can be provided in that this method can be satisfactorily applied even when an infrared ray-opaque silicon sample is to be measured. A quantity of oxygen contained in the silicon sample can be remarkably easily, speedily and accurately measured and this method is very suitable for the use as a simple industrial control analysis. The method of the invention comprises the addition of various measures to the conventional method, such as using a double graphite crucible consisting of an outside graphite crucible, of which the temperautre can be directly adjusted by electrifying to generate heat, and an inside graphite crucible provided in said outside graphite crucible, of which the temperature is indirectly adjusted (since an electric current flows hardly therethrough) for heating and melting the silicon sample to extract oxygen contained in the silicion sample in form of gases combined with carbon. Not only the double graphite crucible itself is degassed before the silicon sample is charged into the inside graphite crucible but also the flux metal is degassed. Furtheron, the temperature of the double graphite crucible during the extraction of gases is controlled in two stages in order to overcome and eliminate various special problems occuring when this method is applied to a silicon sample which is subjected to a melting type gas extraction analysis which has been used conventionally as a method of analyzing gases in a metal.

## Claims

1. A method of measuring the quantity of oxygen contained in a silicon sample comprising
(i) heating at first a double graphite crucible (C) consisting of an outside graphite crucible (9) of which the temperature is directly controlled by passage therethrough of an electrical current to generate heat and an inside graphite crucible (10) provided in said outside graphite crucible of which the temperature is indirectly controlled at a predetermined high temperature to degas said double graphite crucible itself;
(ii) adjusting the temperature of said double graphite crucible at a predetermined value and charging a flux metal (M) into said inside graphite crucible to degas said flux metal;
(iii) adjusting the temperature of said double graphite crucible at values near the melting point of silicon and charging a silicon sample (S) into said inside graphite crucible to melt said silicon sample in the flux metal and then adjusting the temperature of said double graphite crucible at values which are relatively higher than the melting point of the silicon to extract oxygen contained in said silicon sample in the form of gases combined with carbon; and
(iv) introducing said extracted gases into a gas-concentration analyzing system to detect the gas concentration, thereby measuring the quantity of oxygen contained in said silicon sample.

2. The method according to claim 1 **characterized in that** the gas to be detected is carbon monoxide.

## Patentansprüche

1. Verfahren zur Messung des in einer Siliciumprobe enthaltenen Sauerstoffgehalts, umfassend
(i) Erhitzen eines ersten Doppelgraphittiegels (C), welcher aus einem Außenseitengraphittiegel (9), dessen Temperatur direkt durch Durchleiten eines elektrischen Stroms durch diesen, um Wärme zu erzeugen, reguliert wird, und einem Innenseitengraphittiegel (10), welcher in dem Außenseitengraphittiegel vorgesehen ist und dessen Temperatur indirekt reguliert wird, besteht, bei einer vorbestimmten hohen Temperatur, um den Doppelgraphittiegel selbst zu entgasen;
(ii) Einstellen der Temperatur des Doppelgraphittiegels auf einen vorbestimmten Wert und Einbringen eines Flußmetalls (M) in den Innenseitengraphittiegel, um das Flußmetall zu entgasen;
(iii) Einstellen der Temperatur des Doppelgraphittiegels auf Werte nahe dem Schmelzpunkt von Silicium und Einbringen einer Siliciumprobe (S) in den Innenseitengraphittiegel, um die Siliciumprobe in dem Flußmetall zu schmelzen, und danach Einstellen der Temperatur des Doppelgraphittiegels auf Werte, die verhältnismäßig höher sind als der Schmelzpunkt des Siliciums, um in der Siliciumprobe enthaltenen Sauerstoff in Form von mit Kohlenstoff gebundenen Gasen zu extrahieren; und
(iv) Einführen der extrahierten Gase in ein Gaskonzentrations-Analysensystem, um die Gaskonzentration zu bestimmen, wodurch der in der Siliciumprobe enthaltene Sauerstoffgehalt gemessen wird.

2. Verfahren nach Anspruch 1, **dadurch gekennzeichnet,** daß das zu bestimmende Gas Kohlenmonoxid ist.

## Revendications

1. Méthode de mesure de la quantité d'oxygène contenue dans un échantillon de silicium comprenant :
(I) le chauffage d'un creuset double en graphite (C) consistant en un creuset externe en graphite (9) dont la température y est directement réglée par le passage d'un courant électrique afin de générer de la chaleur et en un creuset interne en graphite (10), pourvu dans ledit creuset externe en graphite, dont la température est réglée indirectement à une valeur déterminée élevée afin de dégazer ledit creuset double en graphite;
(II) le réglage de la température dudit creuset double en graphite à une valeur déterminée et le chargement d'un métal fondant (M) dans ledit creuset interne en graphite afin de dégazer ledit métal fondant;
(III) le réglage de la température dudit creuset double en graphite à des valeurs proches du point de fusion du silicium et le chargement d'un échantillon de silicium (S) dans ledit creuset interne en graphite afin de fondre ledit échantillon de silicium dans le métal fondant et ensuite le réglage de la température dudit creuset double en graphite à des valeurs qui sont relativement supérieures au point de fusion du silicium afin d'extraire l'oxygène contenu dans ledit échantillon de silicium sous forme de gaz combinés avec du carbone; et
(IV) l'introduction desdits gaz extraits dans un système d'analyse de concentration de gaz pour détecter la concentration de gaz et de ce fait mesurer la quantité d'oxygène contenue dans ledit échantillon de silicium.

2. Procédé selon la revendication 1, caractérisé en ce que le gaz devant être détecté est du monoxyde de carbone.
